# EUROPEAN PATENT SPECIFICATION

(11) **EP 3 302 397 B1**
(45) Date of publication and mention of the grant of the patent: **03.01.2024**
(21) Application number: 16726593.3
(22) Date of filing: 02.06.2016
(51) Int. Cl.: A61K 35/35, A61J 1/05, A61M 5/31, C12M 1/26, A61M 5/178, A61K 35/28

(54) **SYRINGE FOR CELL ISOLATION**
SPRITZE ZUR ZELLISOLIERUNG
SERINGUE POUR L'ISOLATION DE CELLULES

(30) Priority: 03.06.2015 EP 15170442
(43) Date of publication of application: 11.04.2018
(73) Proprietor: Educell D.O.O., 1236 Trzin (SI)
(72) Inventor: VEBER, Matija, 1236 Trzin (SI); BARLIC, Ariana, 1236 Trzin (SI); KNEZEVIC DR., Miomir, 1236 Trzin (SI); STRBAD, Marko, 1236 Trzin (SI); GIRANDON, Lenart, 1236 Trzin (SI); DOBRAVC, Janja, 1236 Trzin (SI)
(74) Representative: Hoefer & Partner Patentanwälte mbB
(86) International application number: PCT/EP2016/062573
(87) International publication number: WO 2016/193401

(56) References cited:
- EP-A1- 0 778 794
- WO-A2-2011/145075
- WO-A2-2012/006587
- DE-A1- 4 132 480
- DE-A1-102011 080 218
- US-A- 4 299 238
- US-A- 5 125 414
- US-A1- 2006 079 834
- US-A1- 2013 012 921
- BIANCHI FRANCESCA ET AL: "A new nonenzymatic method and device to obtain a fat tissue derivative highly enriched in pericyte-like elements by mild mechanical forces from human lipoaspirates.", CELL TRANSPLANTATION 2013, vol. 22, no. 11, 2013, pages 2063-2077, XP002752470, ISSN: 1555-3892
- GIMBLE J M ET AL: "DIFFERENTIATION POTENTIAL OF ADIPOSE DERIVED ADULT STEM (ADAS) CELLS", CURRENT TOPICS IN DEVELOPMENTAL BIOLOGY, ACADEMIC PRESS, NEW YORK, NY, US, vol. 58, 1 January 2003 (2003-01-01), pages 137-160, XP009034649, ISSN: 0070-2153, DOI: 10.1016/S0070-2153(03)58005-X
- LIN K ET AL: "Characterization of adipose tissue-derived cells isolated with the Celution (TM) system", CYTOTHERAPY, ISIS MEDICAL MEDIA, OXFORD, GB, vol. 10, no. 4, 1 January 2008 (2008-01-01), pages 417-426, XP009106530, ISSN: 1465-3249, DOI: 10.1080/14653240801982979
- PHILIPPE BOURIN ET AL: "Stromal cells from the adipose tissue-derived stromal vascular fraction and culture expanded adipose tissue-derived stromal/stem cells: a joint statement of the International Federation for Adipose Therapeutics and Science (IFATS) and the International Society for Cellular Therapy (ISCT)", CYTOTHERAPY, vol. 15, no. 6, 1 June 2013 (2013-06-01), pages 641-648, XP055143289, ISSN: 1465-3249, DOI: 10.1016/j.jcyt.2013.02.006

## Description

The present application is concerned with a syringe that is useful for isolating cells such as stem cells from tissue.

### BACKGROUND OF THE INVENTION

Mesenchymal stem cells (MSCs) have been studied as cell source for regenerative medicine in different contests. Over the last decade, MSCs have emerged as one of the most rapidly growing areas of clinical cell therapy in regenerative medicine. MSCs cells are able to self-renew with a high growth rate and possess multi-potent differentiation properties. Beyond from bone marrow, MSCs can be obtained from the dental pulp, fetal membranes of term placenta, and adipose tissue.

The use of adipose tissue-derived progenitors as a therapeutic agent has grown substantially in the past decade and has sparked the growth of a new research field and industry worldwide (e.g. Gimble JM et al. Curr. Top. Dev. Biol., 58, 137, 2003; Timper K et al. Biochem. Biophys. Res. Commun., 341, 1135, 2006; Tremolada C et al. Cell Transplant., 19, 1217 2010). Adipose-derived stem cells (ASCs) exhibit phenotypic and gene expression profiles similar to MSCs obtained from bone marrow and other alternative sources, can be expanded in culture for extended periods and have become an accessible source of multipotent stromal cells and, therefore, promising tool for regenerative therapies (e.g. Zuk PA et al. Mol Biol Cell.,13, 4279, 2002).

Human subjects have abundant subcutaneous fat deposits and ASCs can easily be isolated, in general by enzymatic digestion of lipoaspirates, thus overcoming the tissue morbidity associated with bone marrow aspiration. Furthermore, the frequency of MSCs in bone marrow is between 1 in 25,000 and 1 in 100,000 cells, whereas ASCs constitute approximately 2% of lipoaspirate cells (e.g. Bianchi F et al. Cell Transplantation, 22, 2063, 2013).

Since late '60s, multiple laboratories have isolated MSCs from adipose tissue, e.g. resected as intact tissue or aspirated using tumescent liposuction. More recently, international standards based on reproducible parameters that promote biological clarifications, best clinical practices and safety to improve efficacious adipose tissue-derived cell therapies have been proposed to set standard protocols and controls (Bourin P et al. Cytotherapy, 15, 641, 2013). However, as a general procedure, minced adipose tissue is digested by one or more compounds like collagenase, dispase, trypsin or related enzymes at 37°C for 30-60 minutes.

Based on this methodological approach, many devices have been commercialized for isolation of ASCs and successive use in regenerative therapy, by proposing different technical solutions for automatization, avoidance of external contamination, process time, cost of consumables, etc.

The basic principle is, first, to centrifuge the aspirated fat tissue after dilution with water or aqueous solution to remove red blood cells and pure oil fraction and obtain the pure fat. Then, the fat fraction is digested by adding an enzyme solution by contemporary incubation at 37°C and shaking to obtain an emulsion where the stromal vascular fraction (SVF) also containing ASCs is present. At the end, the emulsion is centrifuged to obtain the fraction with stem cells, which can be further washed or is directly used for therapy.

Different instruments are available in commerce based on this principle. Not exhaustive examples are Celution^{®} 800/CRS System by Cytori, Cha-Station^{™} by Inphronics, Icellator Cell Isolation System^{®} by Tissue Genesis, Sceldis^{®} by Medica and UNiStation^{™} by NeoGenesis.

In general the known methods comprise the following steps of a) washing of lipoaspirate and separation (centrifugation) of the three phases, namely red blood layer, pure fat and free oil; b) discharging the phases, in particular the pure fat to another syringe; c) enzymatic digestion of the pure fat; d) centrifugation and separation of the ASC rich fraction; and e) discharging and collection of the ASC fraction in another syringe or container for further treatments.

In methods as used in the prior art, the different fractions in the syringe are discharged one by one through the hole at the bottom of the syringe by pushing the plunger. By means of that, the different fractions can be cross-contaminated during the discharging phase. In fact, the modulation of pushing pressure on the plunger and the way how the syringe is kept are highly crucial to avoid, or limit remarkably, a mixing between two contiguous layers on the contact surface. The probability that cross-contamination occurs to a lower or higher extent is high.

Moreover, these instruments are highly customized in their components and disposables (i.e. type of syringe). It means that the final user has to buy the instrument and, above all, the customized disposables even if other equipment, typically centrifuge but also shaking incubator, are already available on his site.

A centrifuge is used to separate cells which thereafter have to be isolated. This can cause problems. By using a fixed angle centrifuge, the pellet forms on the side of the conical end of the syringe and thus cannot be directly pushed out without extensive contamination with the majority of liquids in the syringe. On the other hand, for some commercially available systems it is necessary to use a swing out rotor centrifuge, which is bigger but also more expensive than standard centrifuges.

US 5 125 414 A discloses a blood sampling device, which contains a slidable piston and a hollow barrel. The slidable piston and hollow barrel are designed to enclose a needle after it was used to sample blood from a patient and it is intended to protect the operator from transmission of infectious diseases. The used needle, which is connected to the slidable piston, is retracted into the hollow barrel of the blood sampling device by action of a spring inside the barrel, which upon release forces the needle into the barrel. The slidable piston of the blood sampling device does not comprise a septum, it does not comprise a handle, and it does not comprise a continuous hollow channel which extends from piston to handle.

US 4 299 238 A discloses a vented piston and push-rod subassembly for use in a syringe barrel. The push-rod type plunger of the disclosed syringe comprises an integrally-formed thumb rest, and is the part, which is handled. This plunger is combined with the piston. In this assembled state, liquid, which can enter the hollow interior of the piston, is separated from the plunger by a liquid-tight seal. Therefore, the disclosed plunger of the syringe does not comprise a hollow channel, which spans a slidable piston and handle.

DE 41 32 480 A1 discloses a device for blood sampling. The device comprises a syringe comprising a barrel and a plunger. The plunger is one piece, which is a hollow cylinder comprising a one-way check valve at the distal end. It is either open at the proximal end, or closed with a cap. The valve allows only entering of liquid in one way, namely from the barrel into the plunger. Therefore, the valve cannot be seen as a septum, which allows liquid transfer in both directions. Furthermore, the plunger does not comprise a hollow channel, which spans a slidable piston and handle.

One object of the present invention is the provision of an instrument that can be used to separate cells, which is easy to handle, causes no or few contamination, and can be adapted to various types of centrifuges or instruments commonly used in laboratories and treatment centers. Furthermore, it is an object to provide an instrument or device, which comprises parts in a disposable and/or sterilizable form or wherein the device in total is sterilizable.

Furthermore, it is an object of the present invention to provide a device for obtaining stem cells in high yields, high quality and at high purity, which can be used for isolation of stem cells, in particular stem cells from adipose tissue.

### DESCRIPTION OF THE INVENTION

The aforementioned objects are achieved and the problems of the prior art are solved by a syringe of the present invention.

The syringe of the present invention comprises a barrel or syringe body with two ends, one proximal end for insertion of a plunger, and a distal end with a tip or orifice, also called connector, that is connectable with a cannula or can be locked with a locking system. A plunger is slidably movable in the barrel. The plunger can be pulled and pushed along inside the barrel, allowing the syringe to take in or expel material. The connector of the syringe can be fitted with a tip, like a hypodermic needle, a nozzle, or tubing to direct the flow into and out of the barrel.

The plunger comprises at least three individual parts that can be assembled to be inserted into the barrel. The individual parts comprise a handle, a piston, and a septum. Handle and piston comprise one or more hollow channels and the handle is connectable to the piston such that at least one channel of the handle and at least one channel of the piston form a continuous channel when assembled. Furthermore, the piston on the side distal to the handle is connectable to a disc-like septum, such as a rubber head. When the parts are assembled they together form the plunger with at least one hollow channel enabling free access to the septum. At least one channel allows to insert a charger/discharger device like a tip, a needle or cannula. When the assembled plunger is inserted into a barrel the septum tightly closes the barrel and can be punctured by a tip like a needle or cannula.

When the barrel is filled with material, such as an isolate, the barrel can be tightly closed on both sides. The upper part of the barrel is closed by the disc-like septum and the tip is closed by a locking system, for example can be closed by a stopper or another closing means.

The barrel can have any shape, preferably it is a cylinder having a circular or oval cross-section. The inner and outer surface of the cylinder is uncritical, it can be smooth or grooved and the form is also uncritical. The barrel can be prepared from any material usually used for preparing syringe barrels, in particular a transparent material, such as glass or a polymer.

The plunger is slidably movable within the barrel, it is form fit insertable into the barrel. The handle and the piston can be made from any material commonly used for this type of devices such as a polymer, metal or glass, in particular a bio-compatible polymer that is inert with regard to tissue, cells and body fluids. Suitable polymers are for example HDPE, PVC, Teflon, ABS, natural rubber or synthetic rubber, or silicon polymers, or a combination thereof.

The handle can have any cross section, such as a circular, oval, rectangular, star-shaped, T-shaped, double T-shaped (train rail like), or X-shaped cross-section. The handle can have a top portion that is overhanging compared to the cross section of the handle, it can for example be disc-like. The handle is used to pull or push the piston.

It is an important feature of the present invention that the handle comprises at least one hollow channel that runs along the length of the handle, it can comprise one or more than one channel. The number of channels in the handle depends on the size of the barrel and the size of a channel. The size of at least one channel is such that a charge/discharge device such as a tip or tubing can be slid or pushed through. When the handle comprises a top part in the form of a disc, this disc has at least one hole that continues as channel. A channel that is adapted for pushing through a tip or tubing has suitably a surface that is smooth enough to allow sliding of such tip or tubing. The tip or tubing can be connectable to a charge/discharge device or transfer device

The arrangement of the channels in the handle is not critical. Thus, any channel can either be arranged eccentrically or centrically or, if there is more than one channel, as a combination thereof. As a not limiting example, there can be one hollow channel having a medium to large size, in the centre of the plunger or in the outer ring. There can also be a series of channels around the center or in any other arrangement. Another option is one channel in the centre and channels surrounding it. If there is more than one channel, the channels can also form patterns like a circle or other geometrical figure, two or more concentric or excentric circles around the centre of the plunger etc. Moreover, the circles or other geometrical figures can be in the inner, or outer, or both parts of the plunger top. The outer arrangement allows a better separation of upper phases (e.g. oil and fat). Moreover, the size of the hollow channels can include a range of different possible diameters.

The piston is connectable with the handle on the proximal side and with the septum on the distal side. The connections are such that when assembled handle, piston and septum can be slid together within the barrel without being disassembled. The piston can be connected with the handle by any connecting means, for example via a threaded connection, by attachment or plug-on or plug-in. In one embodiment, the piston is made of a polymer, preferably the same polymer as the handle. The piston does not have to seal the barrel as this function is fulfilled by the septum.

The septum can be made from any material usually used therefore, i.e. a flexible resilient material such as rubber. The form of the septum is such that it form fits into the barrel and closes the barrel. The septum can be pierced by a tip like a needle or cannula and closes after the needle or cannula has been withdrawn.

The distal end of the syringe can be a tip that can be connected to a charge/discharge device or transfer device like a cannula or a tubing or can have closure means, like a stopcock or a stopper to allow the tip to be closed and opened on demand. A charge/discharge device or transfer device is any device that allows transferring material, such as fluids or cells, and comprises a tip like part that can be introduced into the septum or can be connected with the distal end of the syringe. The charge/discharge device can comprise in addition to the tip a transfer part, such as a tubing. In one embodiment a standardized system of small-scale fluid fittings is provided, such as a Luer lock system, to allow for leak-free connections between the tip of the syringe and a cannula or tubing. Male-taper fitting and its mating female part on medical and laboratory instruments are well known, including hypodermic syringe tips and needles or stopcocks and needles. It is also possible to provide a tip with a thread or a bayonet closure as connector means. The connector or tip at the distal end of the syringe can be used to expel liquid out of or to draw liquid into the lower part of the syringe. In another embodiment, the tip is closed with a stopper.

The at least one continuous channel in the assembled plunger allows a charger/discharger device to be inserted. A charger/discharger device, comprising a tip, such as a cannula or tubing or any useful means for piercing a septum, is used to introduce substances like enzymes, washing solution, etc. that are for example used in the process of digestion and isolation of ASCs, and/or to remove or withdraw one or more layers or part thereof, i.e. part of the material in the syringe like cells or fat. Commonly a charger/discharger device useful for the present invention comprises a tip, to be pierced through the septum, connected to a tube. Such devices can be hollow needles, hollow sticks, a tip connected to a flexible tube or a rigid tip as part of a flexible tube.

If more than one hollow channel is provided, more than one tip and/or tubing can be used to draw material into the barrel and/or to expel material out of the barrel. Thus, in one embodiment, two channels are provided, one for drawing liquid, such as an enzyme solution, from a reservoir into the barrel and another one for withdrawing cells out of the barrel into a container. For each liquid or material to be fed or withdrawn a separate tip and/or a separate charge/discharge device like a tubing with a tip can be used. The transport part of a charge/discharge device suitably is made from a material that is inert and optionally cell compatible and can be tightly connected with a tip for transporting liquid or cells. It can be fitted on a tip, like a needle or cannula that is used for piercing the septum or it can have a rigid part that can be directly used for piercing.

It is possible to use only one charge/discharge device for feeding and withdrawing or to use a separate charge discharge device for any feeding or withdrawing action. It is also possible to use only one tip and change the tubings.

The innovative functional device of the present invention allows modulation and fine tuning of material and the liquids to be introduced to and removed from the syringe. The barrel can be filled with isolate directly and the plunger can be inserted thereafter or the barrel can be filled with isolate via the distal end tip or via a charger/discharger device.

For charging or discharging the barrel positive or negative pressure can be applied manually by moving the plunger or externally via another syringe or pump means connected via the charge/discharge device. In one embodiment, pressure is applied by manually moving the plunger upwards or downwards.

A pumping system can be another syringe or a pump and can be used for feeding and/or withdrawing material or liquids and it can be connected to one or more charge/discharge devices. The pump can be any type of pump, such as a peristaltic pump, vacuum pump, micropump or syringe pump, preferably it is a peristaltic pump or micro pump. The pump can either apply positive pressure in order to transfer material such as liquids from a reservoir into a barrel of a syringe or negative pressure in order to transfer liquids from a syringe into a reservoir or container. Tips, tubings or needles can be connected to a tube of the pumping system. The pump allows on/off setting and modulation of operational speed (volume per time unit). Such a combined system can be maneuvered easily and with high precision and is also part of the present invention.

A pumping system can for example be connected to a reservoir for liquids and/or material that shall be fed to the syringe via a tube wherein the reservoir can be used as a liquid tank. A pumping system can also be used for material transfer by connecting the syringe of the present invention with a reservoir for storage of or container for isolated stem cells or other material.

The whole syringe or parts thereof, such as charge/discharge device, tips, tubing, septum, can be disposable or can be made from sterilizable material. Each hollow stick, hollow needle and tubing can be dedicated only to one single operational step or substance.

Moreover, the syringe can be placed on a fixed support during the insertion or removal of liquids, thus eliminating completely human intervention (touching) of the operator on the syringe, thereby avoiding external contamination and cross-contamination between centrifuged and separated layers. Thus, a system comprising a syringe and a support for the syringe, optionally combined with a pumping system comprising a pump, at least one reservoir or container and transporting means like tubings, is also part of the present invention.

With the syringe of the present invention stem cells can be isolated in high yield and at high purity.

Surprisingly it was found that the syringe of the present invention can be easily adapted to any centrifuge. Thus the syringe of the present invention can be used in any type of centrifuge and, even more in general, any commercially available instrument for separating stem cells. There is no need to acquire a customized system for digestion and isolation, but instruments already on site, or commercially available standard instruments can be used. This aspect is particularly important for the compatibility with fixed angle centrifuges, the design of which is *per se* a limiting factor not allowing sufficient flexibility in the operational parameters.

The syringe of the present invention, vice versa, allows for using the fixed angle centrifuges in their standard settings. The liquids can be easily removed from the top of a syringe without disturbing the pellet of cells.

In the following a syringe and a syringe system are described in more detail by referring to the drawings wherein

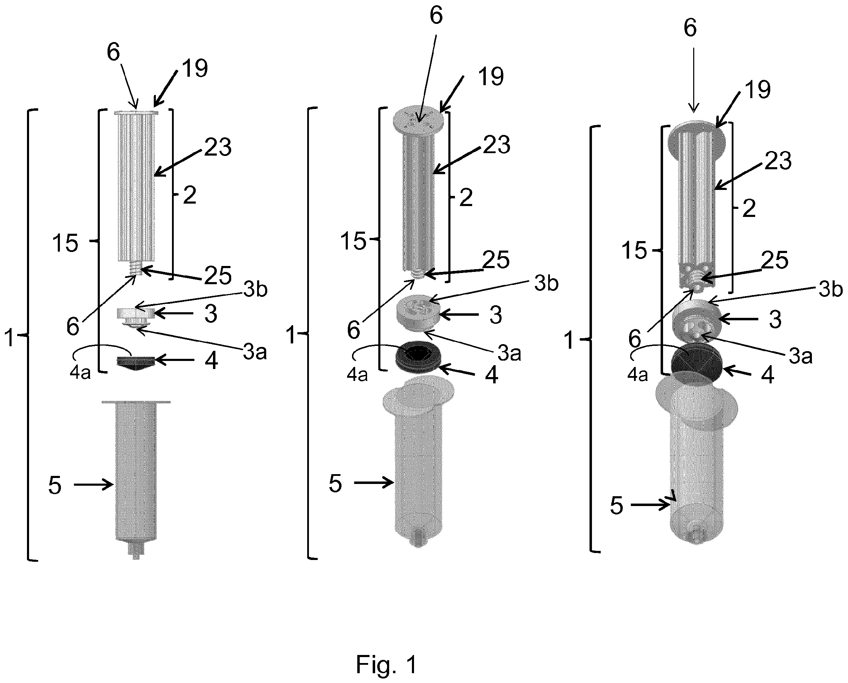

Fig. 1 shows an embodiment of the syringe of the present invention. The syringe 1 comprises a removable plunger 15 and a syringe barrel 5. The removable plunger 15 comprises a handle 2, a piston 3 and a rubber head 4. The removable plunger 15 is form-fit connectable to the syringe barrel 5. The removable plunger 15 can be made of any suitable material, for example a polymer selected from HDPE, PVC, Teflon, ABS, natural rubber or synthetic rubber, or silicon polymers, or a combination thereof.

The handle 2 comprises a top 19, a cylinder 23 and a bottom connection element 25 to pull or push the handle, which has a double T shape cross-section, wherein the top 19 is overhanging compared to the cross section of the cylinder 23.

The handle 2 further comprises five hollow channels 6, one in the centre and four in the ribs of each T, wherein the channels 6 permeate the handle completely. The connection element 25 at the bottom comprises a thread or a bayonet closure.

Piston 3 comprises a connection element on the top side 3b and a connection element on the bottom side 3a. The piston 3 comprises five hollow channels. The eccentric channels of the piston 3 are broadened compared to the channels in the handle, in order to form continuous tubes between both elements even if both elements are not completely assembled. The cross-section of the piston has a circular shape. The piston 3 has a diameter, which is form-fit within the syringe barrel 5, in order to be slidably moved by a user but to be tight enough for centrifugation steps.

The rubber head 4 is form-fit connectable to the piston and also form fit connectable to the syringe barrel 5 for reasons mentioned above. The rubber head 4 comprises a connection element 4a. The rubber head is conically shaped. Furthermore, the rubber head 5 acts as a septum, i.e. it can easily be pierced through by a hollow needle or a hollow stick and the injection site closes after the removal of the hollow needle or hollow stick. This is of particular importance, since otherwise valuable liquids could be lost during centrifugation steps.

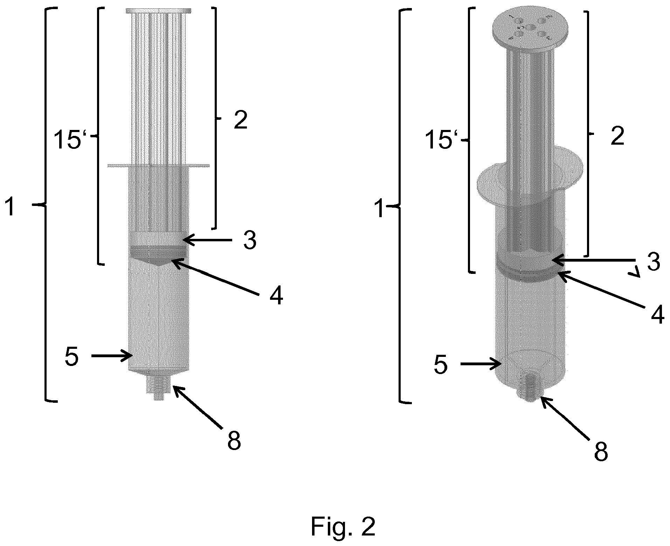

Fig. 2 shows the assembled syringe 1. The assembled plunger 15' comprises a handle 2, a piston 3 and a rubber head 4, whereby the piston 3 and the handle 2 are connectable via the connection elements 25 and 3b; and the piston 3 and the rubber head 4 are connectable via the connection elements 3a and 4a. When the piston 3 and the handle 2 are connected the channels of both elements form continuous tubes together in order to provide access to the liquids of the syringe. For complete assembly of the plunger 15' the rubber head is placed onto the piston 3. The assembled plunger 15' is form-fit connectable to the syringe barrel 5. The syringe barrel 5 comprises a connection element 8 which can be closed by a stopper 30 or can be connected to a needle or hollow stick e.g. in order to collect lipoaspirate.

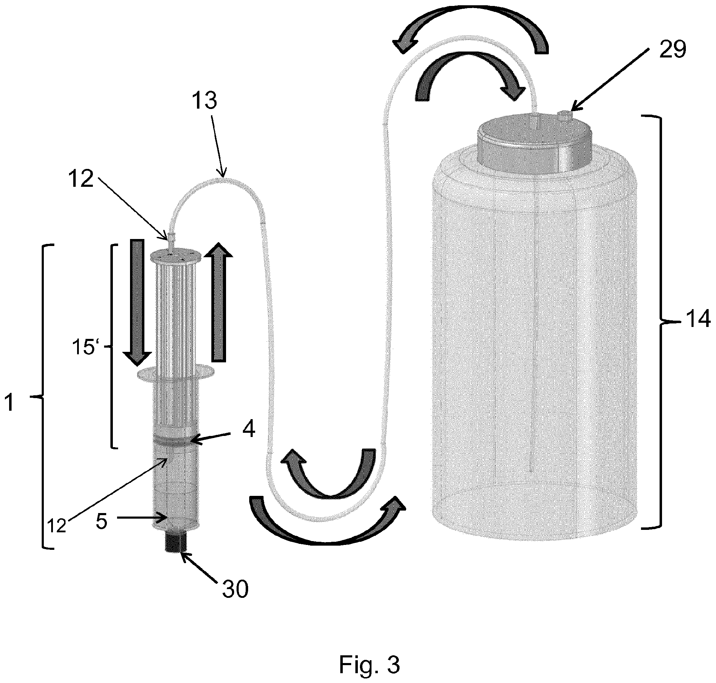

Fig. 3 shows a syringe system of the present invention, comprising an assembly of syringe 1 and a reservoir 14. A hollow needle or a hollow stick 12 connected to a tube 13 are connected to a reservoir 14. The rubber head 4 of the assembled plunger 15' can be pierced by the hollow needle or a hollow 12 stick for the removal or the addition of liquids whereby the syringe barrel 5 is closed by a stopper 30. The liquids can be either removed or added from or to the syringe if a positive or a negative pressure is applied such as by manually moving the plunger upwards or downwards. The material of the tube 13 has to be biocompatible, for example is made from PTFE.

The removed material is fed to the reservoir via tube 13. The reservoir comprises a 0.22 um air filter 29, through which the pressure inside the reservoir equilibrate while filling/removing the liquid.

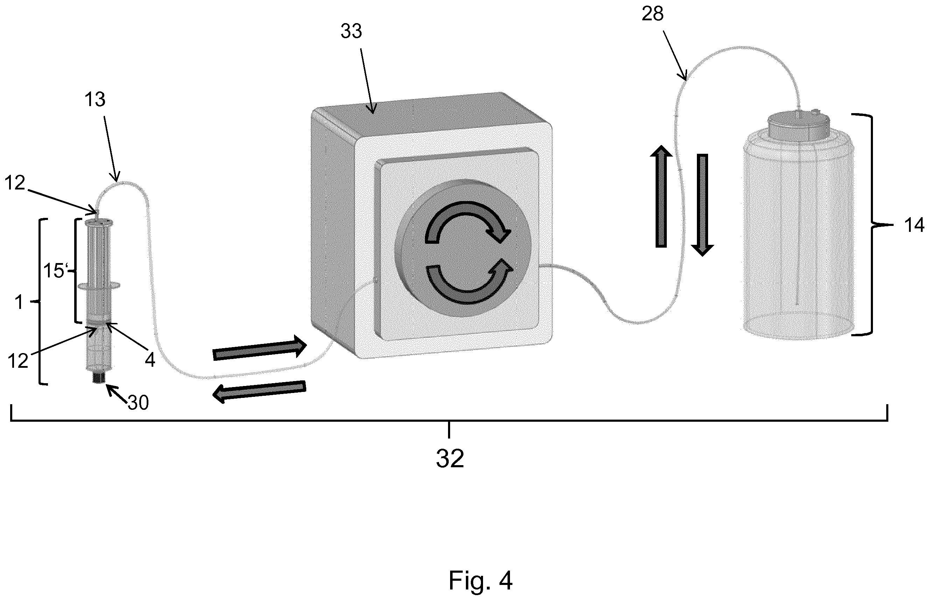

Fig. 4 shows a syringe system 32 of the present invention, comprising a syringe 1 and a hollow needle 12 which is pierced through the rubber head 4 and which is connected to a tube 13, wherein the tube is connected to a pump 33. The pump is connected to a reservoir via a tube 28. The syringe barrel 5 is closed by a liquid stopper 30. The material of the tubes 13 and 28 is biocompatible, or example is made of PTFE.

The pump 33 is a peristaltic pump or micro pump. The pump can either apply positive pressure in order to transfer liquids from the reservoir 14 into the syringe 1 or negative pressure in order to transfer liquids from the syringe 1 into the reservoir 14.

The syringe and the syringe system of the present invention can be used to isolate stem cells in a gentle and convenient manner. Therefore, another aspect of the present invention is a method for isolating stem cells from tissue, such as adipose tissue, which comprises:
a) charging a barrel of a syringe of the present invention with lipoaspirate obtained from a patient;
b) optionally washing the lipoaspirate in the barrel;
c) centrifuging the syringe including the lipoaspirate for separating a fat phase comprising stem cells;
d) processing the fat phase to isolate stem cells;
e) optionally centrifuging the processed fat phase and
f) isolating a stem cell enriched fraction.

Lipoaspirate can be obtained from adipose tissue. Other sources for stem cells can be used as well. The lipoaspirate comprises cells, like stem cells and red blood cells, and liquids like fat and oil. The lipoaspirate is filled in a barrel and processed. It can optionally be washed by feeding a washing solution and withdrawing it for example via charge/discharge device.

The barrel including the lipoaspirate is centrifuged to separate red blood cells and oil from a fat phase. The layers with red blood cells and oil can be withdrawn either via the distal end or via a charge/discharge device inserted through a channel in the handle/piston and through the septum in the syringe of the present invention. It is also possible to withdraw the stem cell comprising fat layer via a charge/discharge device and process it in another device. In both cases it is possible to withdraw a layer without or hardly without contamination by the other layer(s).

Any centrifugation step can be carried out by any instrument known in the art for separating such type of material, for example a fixed-angle centrifuge or a swing-out rotor centrifuge, preferably by a swing-out rotor centrifuge. It is one of the advantages of the syringe of the present invention, that centrifugation is not restricted to a specific type of centrifuge.

The fat phase is then processed to isolate stem cells. The fat phase can either be processed in the same barrel or can be transferred into a fresh container, such as a syringe barrel or another device for further processing. As the withdrawal of phases from a syringe of the present invention is easily achieved via one of the channels provided, transfer into a fresh container might be useful to get rid of contaminating material. Processing of the fat phase can be done as is well known in the art, for example by adding an enzymatic solution for digestion, such as a collagenase solution. Thereby the stem cells are released and can be separated by another centrifugation step. The use of a second syringe is particularly useful when another centrifugation step is applied.

A fraction enriched with stem cells can then be recovered.

The method of the present invention allows obtaining stem cells from tissue in a straightforward manner, with no or little risk of contamination and in a very gentle manner that prevents damage from the cells.

Stem cells can be isolated from tissue according to the method of the present invention using a syringe or a syringe system or a device according to the present invention. The method comprises steps a) to f) as outlined above.

A barrel of a syringe of the present invention can be charged either via the distal or the proximal end with a sample like a lipoaspirate. The barrel then can be tightly closed by inserting a stopper on the distal end and inserting the plunger including a septum on the proximal end.

The sample can be washed in the barrel for example by introducing washing solution and withdrawing it via one or more of the channels by using a charge/discharge device optionally together with a pump.

The sample then is centrifuged in the syringe to separate layers like a cell comprising layer and an oil layer, for example for separating a fat phase comprising stem cells from an oil layer or by separating layers with different cells.

The syringe of the present invention allows withdrawing whichever layer without any or hardly any contamination by other layers by directly guiding the charge/discharge device to the layer to be withdrawn without disturbing other layers via at least one of the channels in the plunger and a tip piercing the septum. Thereby a relatively pure layer can be obtained. Thus, a fat phase that has been separated from a lipoaspirate can be either directly withdrawn into another barrel and can be processed there or any other layer except the fat layer can be withdrawn and the fat phase can be directly processed in the barrel where it has been separated. By processing the fat phase essentially without contaminating other layers a highly enriched stem cell preparation can be obtained.

The processed preparation can, if necessary, be centrifuged, again directly in the barrel of the syringe and a stem cell enriched fraction can be obtained.

The invention will be now described by reference to the following non-limiting examples.

### EXAMPLES

### Example 1 (for comparison)

Stem cells were isolated by using a commercially available device. Commercially available pumps, reservoirs, containers, tubings etc. were used. The devices used for the tests were the same for all samples.

A fraction containing ASCs was prepared strictly in compliance with the instructions reported by the supplier of the device.

The following main steps were performed:
a. washing of lipoaspirate and separation by centrifugation of red blood layer, fat and free oil;
b. discharging the three phases one by one,
c. transferring the fat phase to another syringe;
d. enzymatic digestion of the fat phase;
e. centrifugation and separation of the ASCs rich fraction; and
f. discharging and collection of the ASCs fraction in another syringe or container for further treatment.

The above operation was repeated by 5 different operators.

After step b., the residual content of red cell and fat oil (contamination) in the pure fat fraction was determined in the fat fraction according to a semi-quantitative evaluation rank (1-absent, 2-very slight, 3- slight, 4-moderate, 5-remarkable). The results are reported in Table 1.

**Table 1. Residual content of red cell and fat oil in the pure fat fraction after step b.**

| Operator | Red cell | Fat oil |
|---|---|---|
| 1 | 2 | 2 |
| 2 | 3 | 3 |
| 3 | 3 | 3 |
| 4 | 2 | 3 |
| 5 | 3 | 2 |

The pure fat fraction is slightly or moderately contaminated. The extent of contamination is dependent on the human variable (operator). Fat oil contamination is, on average, lower than red cell contamination. The discharging procedure allows for retaining part of the fat fraction in the syringe together with the fat oil, thus reducing the extent of contamination.

Analogously, the same scale was adopted for the determination of contamination in the digested phase with stem cells after step e.. In this case, a different volume of stem cell fraction was removed at step d. in different trials. The aim was to check the contamination extent and at different yield of final product. The yield is expressed as ratio between effective collected volume of stem cell fraction versus total volume of stem cell fraction in the syringe at the end of step d.. The results are reported in Table 2.

**Table 2. Contamination level at different yield at the end of step d.**

| Yield (%) | Contamination level |
|---|---|
| 100 | 5 |
| 90 | 4 |
| 80 | 2 |
| 70 | 0 |

There is a direct relationship between yield and level of contamination. The collected volume (yield) must be lower than 70-80% to have a stem cell fraction not contaminated or with a slight, acceptable, level of contamination.

### Example 2 (according to the present invention)

25 mL fat were aspirated from subcutaneous adipose tissue by means of the syringe of the present invention. The distal end of the syringe was closed with a stopper. A washing solution of 5 mL saline solution was added. The syringe was shaken for 2 minutes at 200 rpm in a shaker and then centrifuged at 1200 rpm for 2 minutes. The two fractions were removed, one by one (in order, fat oil and red blood cells), by aspiration with tubes connected to a micro-pump. The contamination in the fat fraction was determined (5 trials with different operators). The results are reported in Table 3.

**Table 3. Red cell and fat oil contamination in pure fat fraction.**

| Operator | Red cell | Fat oil |
|---|---|---|
| 1 | 0 | 1 |
| 2 | 0 | 1 |
| 3 | 1 | 1 |
| 4 | 0 | 0 |
| 5 | 1 | 0 |

Compared to Example 1, the level of contamination is much lower by using the syringe of the present invention. Moreover, the variability among operators is also lower.

Fat fraction and 0,5 PZ U/mL collagenase solution were then transferred to another syringe of the present invention and digested in a shaking incubator at 37°C for 30 minutes. The suspension was centrifuged at 800 rpm for 5 minutes. The fractions without stem cells and that with stem cells were then withdrawn one by one by aspiration with tubes connected to a micro-pump.

The level of contamination versus the yield, expressed as in Example 1, was also determined. To this aim, small volumes of the fraction with stem cell were also withdrawn together with the fraction without stem cell when the latter one was aspired. The results are reported in Table 4.

**Table 4. Contamination level at different yield.**

| Yield (%) | Contamination level |
|---|---|
| 100 | 2 |
| 90 | 1 |
| 80 | 0 |
| 70 | 0 |

There is a direct relationship between yield and level of contamination. However, the level of contamination is much lower at the same yield than in Example 1. The collected volume (yield) of stem cell fraction is substantially not contaminated already at 90% yield.

### Example 3

Different syringes of the prior art and syringes of the present invention with the channeled plunger were placed in different types and models of fixed angle centrifuge to determine their compatibility. The results are reported in Table 5.

**Table 5. Compatibility of syringes of the prior art and syringes of the present invention with channeled plunger with different types and models of fixed angle centrifuge.**

| Centrifuge | Syringe of prior art | Syringe with channeled plunger |
|---|---|---|
| Corning^{®} LSE^{™} Compact Centrifuge | NO | YES |
| VWR^{®} Clinical 200 Large Capacity Centrifuge | NO | YES |
| Compact Research Centrifuge Z206-A-230V | NO | YES |

## Claims

1. A syringe comprising:
a barrel having a proximal end for insertion of a plunger and a distal end with a tip, and a plunger slidably movable in the barrel, wherein the plunger comprises a handle with at least one hollow channel, a piston with at least one hollow channel, and a septum, wherein handle and piston when assembled form at least one continuous channel.

2. A syringe according to claim 1, wherein the plunger is made of metal or a plastic material, selected from HDPE, PVC, Teflon, ABS, natural rubber, synthetic rubber or silicone polymers, or a combination thereof.

3. A syringe according to one of claims 1-2, wherein the at least one hollow channel in the handle and in the piston are positioned centrically and/or eccentrically.

4. A syringe according to one of claims 1-3, wherein the handle has a rectangular, an X-shaped, double T-shaped, star-shaped or circular shaped cross-section.

5. A syringe according to one of claims 1-4, wherein the handle has a smooth or grooved external side surface.

6. A syringe according to one of claims 1-5, wherein the septum is a rubber disc.

7. A syringe system comprising a syringe according to one of claims 1-6, and a charge/discharge device that is insertable in at least one channel formed in the handle and piston and which optionally is connectable to a tubing.

8. A syringe system according to claim 7 comprising additionally a pump.

9. A syringe system according to one of claims 7-8, wherein the syringe system comprises at least one reservoir and/or one container.

10. A syringe system according to one of claims 7-9, wherein the pump is a manual pump, a vacuum pump, a micropump, a peristaltic pump or a syringe pump.

11. A device for processing a lipoaspirate comprising a syringe according to one of claims 1 to 6, a tip or cannula that is insertable in one channel formed in the handle and piston, a tubing connected with the tip or cannula, a pump system to charge or discharge liquids to or from the syringe through the septum, and at least one reservoir and/or container.

12. A method for isolating stem cells from tissue, comprising:
charging a barrel of a syringe of anyone of claims 1 to 6 with lipoaspirate obtained from a patient;
optionally washing the lipoaspirate in the barrel;
centrifuging the syringe including the lipoaspirate for separating a fat phase comprising stem cells;
processing the fat phase to isolate stem cells;
optionally centrifuging the processed fat phase and
isolating a stem cell enriched fraction.

13. A method according to claim 12, wherein the centrifugation step is carried out with a fixed-angle or a swing-out rotor centrifuge.

14. A method according to one of claims 12-13, wherein the tissue is adipose tissue.

15. A method according to one of claims 13 or 14, wherein the fat phase is processed by adding an enzyme solution for digestion.

## Patentansprüche

1. Spritze umfassend:
einen Zylinder mit einem proximalen Ende zum Einführen eines Stempels und einem distalen Ende mit einem Konus, und einen in dem Zylinder verschiebbaren Stempel, wobei der Stempel einen Griff mit mindestens einem Hohlkanal, einen Kolben mit mindestens einem Hohlkanal und ein Septum umfasst, wobei Griff und Kolben, wenn zusammengefügt, mindestens einen kontinuierlichen Kanal formen.

2. Spritze nach Anspruch 1, wobei der Stempel aus Metall oder einem Kunststoffmaterial, ausgewählt aus HDPE, PVC, Teflon, ABS, Naturkautschuk, synthetischem Kautschuk oder Silikonpolymeren, oder einer Kombination davon gefertigt ist.

3. Spritze nach einem der Ansprüche 1 und 2, wobei der mindestens eine Hohlkanal im Griff und im Kolben zentrisch und/oder exzentrisch angeordnet ist.

4. Spritze nach einem der Ansprüche 1 bis 3, wobei der Griff einen rechteckigen, einen X-förmigen, einen Doppel-T-förmigen, sternförmigen oder kreisförmigen Querschnitt umfasst.

5. Spritze nach einem der Ansprüche 1 bis 4, wobei der Griff eine glatte oder gerillte äußere Seitenoberfläche umfasst.

6. Spritze nach einem der Ansprüche 1 bis 5, wobei das Septum eine Kautschukscheibe ist.

7. Spritzensystem umfassend eine Spritze nach einem der Ansprüche 1 bis 6 und eine Befüll-/Auslassvorrichtung, die in mindestens einen im Griff und im Kolben geformten Kanal einführbar ist und die optional mit einem Schlauch verbindbar ist.

8. Spritzensystem nach Anspruch 7 umfassend zusätzlich eine Pumpe.

9. Spritzensystem nach einem der Ansprüche 7 und 8, wobei das Spritzensystem mindestens ein Reservoir und/oder einen Behälter aufweist.

10. Spritzensystem nach einem der Ansprüche 7 bis 9, wobei die Pumpe eine Handpumpe, eine Vakuumpumpe, eine Mikropumpe, eine Peristaltikpumpe oder eine Spritzenpumpe ist.

11. Vorrichtung zur Verarbeitung eines Lipoaspirats umfassend eine Spritze nach einem der Ansprüche 1 bis 6, einen Konus oder Kanüle, die in einen im Griff und im Kolben geformten Kanal einführbar ist, einen mit dem Konus oder Kanüle verbundenen Schlauch, ein Pumpensystem zum Einfüllen oder Auslassen von Flüssigkeiten in die oder aus der Spritze durch das Septum und mindestens ein Reservoir und/oder einen Behälter.

12. Verfahren zur Isolierung von Stammzellen aus Gewebe, umfassend:
Befüllen eines Zylinders einer Spritze nach einem der Ansprüche 1 bis 6 mit einem von einem Patienten erhaltenen Lipoaspirat;
vorzugsweise Waschen des Lipoaspirats in dem Zylinder;
Zentrifugieren der das Lipoaspirat enthaltenden Spritze zum Trennen einer Stammzelle umfassenden Fettphase;
Verarbeiten der Fettphase, um Stammzellen zu isolieren;
vorzugsweise Zentrifugieren der verarbeiteten Fettphase und
Isolieren einer mit Stammzellen angereicherten Fraktion.

13. Verfahren nach Anspruch 12, wobei der Zentrifugationsschritt mit einer Fixwinkel- oder einer Ausschwingrotorzentrifuge durchgeführt wird.

14. Verfahren nach einem der Ansprüche 12 und 13, wobei das Gewebe Fettgewebe ist.

15. Verfahren nach einem der Ansprüche 13 oder 14, wobei die Fettphase durch Hinzufügen einer Enzymlösung zur Verdauung verarbeitet wird.

## Revendications

1. Seringue comprenant :
un cylindre à fonction de corps comportant une extrémité proximale pour l'insertion d'un plongeur et une extrémité distale munie d'une pointe à fonction d'embout, et un plongeur pouvant être déplacé de façon coulissante à l'intérieur du cylindre à fonction de corps, dans laquelle le plongeur comprend un élément de préhension muni d'au moins un canal creux, un piston muni d'au moins un canal creux et un septum, dans laquelle le moyen de préhension et le piston, lorsqu'ils sont assemblés, forment au moins un canal continu.

2. Seringue selon la revendication 1, dans laquelle le plongeur est réalisé en un matériau de métal ou de matière plastique choisi parmi le polyéthylène haute densité ou HDPE, le chlorure de polyvinyle ou PVC, le téflon, l'acrylonitrile butadiène styrène ou ABS, le caoutchouc naturel, le caoutchouc synthétique ou les polymères à base de silicone, ou une combinaison de ceux-ci.

3. Seringue selon l'une quelconque des revendications 1 et 2, dans laquelle l'au moins un canal creux dans le moyen de préhension et l'au moins un canal creux dans le piston sont positionnés de façon centrée et/ou de façon excentrée.

4. Seringue selon l'une quelconque des revendications 1 à 3, dans laquelle le moyen de préhension présente une section en coupe transversale de forme rectangulaire, en forme de X, en forme de T double, en forme d'étoile ou de forme circulaire.

5. Seringue selon l'une quelconque des revendications 1 à 4, dans laquelle le moyen de préhension comporte une surface latérale externe lisse ou rainurée.

6. Seringue selon l'une quelconque des revendications 1 à 5, dans laquelle le septum est un disque en caoutchouc.

7. Système de seringue comprenant une seringue selon l'une quelconque des revendications 1 à 6 et un dispositif de charge/décharge qui peut être inséré dans au moins un canal formé dans le moyen de préhension et dans le piston et qui, en option, peut être connecté à un tubage.

8. Système de seringue selon la revendication 7, comprenant de façon additionnelle une pompe.

9. Système de seringue selon l'une quelconque des revendications 7 et 8, dans lequel le système de seringue comprend au moins un réservoir et/ou au moins un moyen de contenance.

10. Système de seringue selon l'une quelconque des revendications 7 à 9, dans lequel la pompe est une pompe manuelle, une pompe à vide, une micro-pompe, une pompe péristaltique ou une pompe à seringue.

11. Dispositif pour traiter un tissu adipeux de l'être humain résultant d'une liposuccion, comprenant une seringue selon l'une quelconque des revendications 1 à 6, une pointe à fonction d'embout ou une canule qui peut être insérée à l'intérieur d'un canal qui est formé dans le moyen de préhension et dans le piston, un tubage qui est connecté à la pointe à fonction d'embout ou à la canule, un système de pompe pour charger ou décharger des liquides sur ou depuis la seringue au travers du septum et au moins un réservoir et/ou au moins un moyen de contenance.

12. Procédé pour isoler des cellules souches vis-à-vis d'un tissu, comprenant :
le chargement d'un cylindre à fonction de corps d'une seringue selon l'une quelconque des revendications 1 à 6 avec du tissu adipeux de l'être humain résultant d'une liposuccion et prélevé sur un patient ;
en option, le lavage du tissu adipeux de l'être humain résultant d'une liposuccion à l'intérieur du cylindre à fonction de corps ;
la centrifugation de la seringue qui inclut le tissu adipeux de l'être humain résultant d'une liposuccion pour séparer une phase lipidique comprenant des cellules souches ;
le traitement de la phase lipidique pour isoler les cellules souches ;
en option, la centrifugation de la phase lipidique traitée ; et
l'isolation d'une fraction enrichie de cellules souches.

13. Procédé selon la revendication 12, dans lequel l'étape de centrifugation est mise en oeuvre à l'aide d'une centrifugeuse à rotor à angle fixe ou d'une centrifugeuse à rotor libre oscillant.

14. Procédé selon l'une quelconque des revendications 12 et 13, dans lequel le tissu est un tissu adipeux.

15. Procédé selon l'une quelconque des revendications 13 ou 14, dans lequel la phase lipidique est traitée par ajout d'une solution enzymatique pour la digestion.
